# FASCICULE DE BREVET EUROPEEN

(11) **EP 2 190 739 B1**
(45) Date de publication et mention de la délivrance du brevet: **02.02.2011**
(21) Numéro de dépôt: 08804290.8
(22) Date de dépôt: 17.09.2008
(51) Int. Cl.: B64D 29/02, B64D 29/06

(54) **CARENAGE AERODYNAMIQUE ARRIERE INFERIEUR POUR DISPOSITIF D'ACCROCHAGE D'UN MOTEUR D'AERONEF**
UNTERE HINTERSEITIGE AERODYNAMISCHE VERKLEIDUNG FÜR EINE FLUGZEUGMOTORBEFESTIGUNGSVORRICHTUNG
LOWER REAR AERODYNAMIC FAIRING FOR AN AIRCRAFT ENGINE ATTACHMENT DEVICE

(30) Priorité: 20.09.2007 FR 0757712
(43) Date de publication de la demande: 02.06.2010
(73) Titulaire: AIRBUS OPERATIONS, 31060 Toulouse (FR)
(72) Inventeur: JOURNADE, Frédéric, F-31100 Toulouse (FR); RENAUD, Eric, F-31480 Brignemont (FR); JALBERT, Delphine, F-31840 Seilh (FR)
(74) Mandataire: Ilgart, Jean-Christophe
(86) Numéro de dépôt international: PCT/EP2008/062335
(87) Numéro de publication internationale: WO 2009/037267

(56) Documents cités:
- US-A- 4 712 750
- US-A1- 2003 201 366

## Description

### DOMAINE TECHNIQUE

La présente invention se rapporte à un carénage aérodynamique arrière inférieur pour dispositif d'accrochage d'un moteur destiné à être interposé entre une voilure d'aéronef et le moteur concerné, ce carénage étant également appelé « bouclier » ou « APF » (de l'anglais « Aft Pylon Fairing »). Il est notamment connu des documents US 2003/201 366 A1 et US-A-4 712 750.

L'invention peut être utilisée sur tout type d'aéronef équipé de turboréacteurs ou de turbopropulseurs.

Ce type de dispositif d'accrochage, également appelé mât d'accrochage ou « EMS » (de l'anglais « Engine Mounting Structure »), permet de suspendre un turbomoteur au-dessous de la voilure de l'aéronef, ou bien de monter ce turbomoteur au-dessus de cette même voilure.

### ETAT DE LA TECHNIQUE ANTERIEURE

Un tel dispositif d'accrochage est en effet prévu pour constituer l'interface de liaison entre un turbomoteur et une voilure de l'aéronef. Il permet de transmettre à la structure de cet aéronef les efforts générés par son turbomoteur associé, et autorise également le cheminement du carburant, des systèmes électriques, hydrauliques, et air entre le moteur et l'aéronef.

Afin d'assurer la transmission des efforts, le dispositif d'accrochage comporte une structure rigide également dénommée structure primaire, souvent du type « caisson », c'est-à-dire formée par l'assemblage de longerons supérieurs et inférieurs et de panneaux latéraux raccordés entre eux par l'intermédiaire de nervures transversales de rigidification.

D'autre part, le dispositif est muni de moyens d'accrochage interposés entre le turbomoteur et la structure rigide, ces moyens comportant globalement deux attaches moteur, ainsi qu'un dispositif de reprise des efforts de poussée générés par le turbomoteur. Dans l'art antérieur, ce dispositif de reprise comprend habituellement deux bielles latérales raccordées d'une part à une partie arrière du carter de soufflante du turbomoteur, et d'autre part à une attache arrière fixée sur le carter central de ce dernier.

De la même façon, le dispositif d'accrochage comporte également une autre série d'attaches constituant un système de montage interposé entre la structure rigide et la voilure de l'aéronef, ce système étant habituellement composé de deux ou trois attaches.

Par ailleurs, le mât est pourvu d'une pluralité de structures secondaires assurant la ségrégation et le maintien des systèmes tout en supportant des éléments de carénage aérodynamique, ces derniers prenant généralement la forme d'assemblages de panneaux rapportés sur les structures. De façon connue de l'homme du métier, les structures secondaires se différencient de la structure rigide par le fait qu'elles ne sont pas destinées à assurer le transfert des efforts provenant du moteur et devant être transmis vers la voilure de l'aéronef.

Parmi les structures secondaires, on compte le carénage aérodynamique arrière inférieur, également dénommé APF, qui dispose d'une pluralité de fonctions parmi lesquelles on note la formation d'une barrière thermique ou anti-feu, et la formation d'une continuité aérodynamique entre la sortie du moteur et le mât d'accrochage.

Le carénage aérodynamique arrière inférieur prend généralement la forme d'un caisson comprenant deux panneaux latéraux assemblés entre eux par des nervures intérieures transversales de rigidification espacées les unes des autres selon une direction longitudinale du carénage, ainsi qu'un plancher de protection thermique. Il est précisé que ce caisson n'est habituellement pas fermé à l'opposé du plancher de protection thermique, à savoir en partie supérieure lorsque le moteur est destiné à être suspendu sous la voilure de l'aéronef, étant donné que c'est à cet endroit qu'il vient se raccorder sur les autres structures du mât.

Le plancher de protection thermique est pourvu d'une surface extérieure destinée à être épousée par un flux primaire du moteur qu'il délimite, alors que les panneaux latéraux sont quant à eux prévus pour être épousés extérieurement par un flux secondaire du moteur, en raison de leur implantation dans le canal annulaire de flux secondaire du moteur, et/ou en sortie de celui-ci.

Dans les solutions de l'art antérieur, le plancher de protection thermique est monté fixement sur les nervures intérieures transversales du caisson au contact desquelles il se trouve, et ses extrémités latérales opposées sont montées fixement respectivement sur les deux panneaux latéraux qui épousent également les nervures transversales.

Dans cette configuration, le plancher de protection thermique est au contact du flux primaire de température très élevée, ce qui le conduit à se déformer fortement par dilatation thermique. Cependant, ses encastrements respectifs dans les nervures intérieures transversales et dans l'extrémité inférieure de chacun des deux panneaux latéraux engendre de fortes contraintes thermomécaniques au sein du plancher et des panneaux latéraux, ce qui est bien évidemment néfaste pour ces éléments.

Il est noté que ce phénomène d'introduction de fortes contraintes thermomécaniques due à l'importante dilatation thermique du plancher est accentuée par le fait que les panneaux latéraux baignent dans le flux secondaire relativement froid, de sorte qu'ils ne subissent que très peu de déformation par dilatation thermique. Néanmoins, ils rencontrent tout de même une déformation sensible provoquée par la mise en contraintes résultant de la dilatation du plancher auquel ils sont directement et rigidement raccordés, ce qui conduit à dégrader leur forme aérodynamique, et qui, d'une façon plus générale, provoque la détérioration de la qualité aérodynamique globale du carénage. Naturellement, une telle dégradation est pénalisante en terme de traînée parasite engendrée.

A ce titre, il est précisé que la qualité aérodynamique du carénage se trouve également dégradée par les déformations localisées du plancher de protection thermique qui ne peut se dilater librement, sans contrainte, en raison de son encastrement dans certains éléments du carénage telles que les nervures intérieures, comme cela a été décrit ci-dessus. Le flux primaire étant un jet très rapide, les déformations localisées rencontrées au niveau du plancher produisent en effet une traînée parasite sensiblement importante.

Enfin, il est noté que les nervures intérieures transversales, qui ne sont elles pas directement épousées par le flux secondaire sensiblement frais en raison de leur localisation à l'intérieur du caisson, peuvent être sensibles à l'apport de chaleur provenant du plancher de protection thermique avec lequel elles sont en contact. Ainsi, pour leur permettre de remplir leur fonction de maintien mécanique des différents éléments du carénage en forme de caisson, il peut être nécessaire d'avoir recours à un surdimensionnement de ces nervures et/ou à l'utilisation, pour leur fabrication, de matériaux coûteux présentant de bonnes propriétés de résistance à la chaleur.

### EXPOSÉ DE L'INVENTION

L'invention a donc pour but de remédier au moins partiellement aux inconvénients mentionnés ci-dessus, relatifs aux réalisations de l'art antérieur.

Pour ce faire, l'invention a pour objet un carénage aérodynamique arrière inférieur pour dispositif d'accrochage d'un moteur destiné à être interposé entre une voilure d'aéronef et le moteur, le carénage formant caisson comprenant deux panneaux latéraux assemblés entre eux par des nervures intérieures transversales de rigidification espacées les unes des autres selon une direction longitudinale du carénage, et comportant en outre un plancher de protection thermique pourvu d'une surface extérieure destinée à être épousée par un flux primaire du moteur, le plancher de protection thermique présentant deux extrémités latérales opposées.

Selon l'invention, le carénage comporte en outre deux voiles longitudinaux de raccordement déportant le plancher de protection thermique des nervures intérieures transversales de rigidification, ces deux voiles longitudinaux disposant chacun d'une première extrémité latérale montée fixement respectivement sur l'une et l'autre des deux extrémités latérales du plancher de protection thermique, et d'une seconde extrémité latérale montée fixement sur les nervures intérieures transversales de rigidification.

L'une des particularités de la présente invention réside dans le fait que ce plancher est à présent déporté des nervures intérieures transversales à l'aide des voiles longitudinaux, étant entendu que ce sont ces mêmes voiles qui assurent indirectement, de préférence à eux seuls, le montage du plancher sur les nervures. En d'autres termes, le plancher n'est plus monté directement sur les nervures, ce qui lui permet avantageusement de se déformer plus librement par dilatation thermique suite à l'importante chaleur dégagée par le flux primaire épousant ce plancher.

Cette configuration originale dans laquelle le plancher est donc sensiblement libre vis-à-vis des nervures intérieures permet de diminuer considérablement les contraintes thermomécaniques subies par le plancher suite à une telle dilatation, par rapport à celles subies dans les réalisations antérieures dans lesquelles le facteur principal d'introduction des contraintes thermomécaniques dans le plancher était constitué par l'encastrement de ce dernier dans les nervures.

A cet égard, étant donné que le plancher est susceptible de se déformer par dilatation thermique en rencontrant moins de contraintes qu'auparavant, les déformations localisées dégradant la qualité aérodynamique de ce plancher sont de ce fait également fortement diminuées. Il en résulte donc une amélioration de la qualité aérodynamique globale du carénage, diminuant sensiblement les effets de traînée parasite et améliorant par la même le rapport performance/consommation de l'aéronef.

De plus, l'ensemble des avantages indiqués ci-dessus sont accentués par le fait qu'il est également préférentiellement supprimé la liaison mécanique rigide directe entre le plancher et les panneaux latéraux, de manière à créer une coupure mécanique s'étendant longitudinalement entre ces éléments, le plancher pouvant donc se dilater sans entraîner les panneaux latéraux.

De préférence, le plancher déporté des nervures intérieures est entièrement dépourvu de contact avec celles-ci, de sorte que la chaleur transmise à ces nervures par le plancher transite d'abord par les voiles longitudinaux. Cela permet à la chaleur de perdre en intensité avant d'atteindre les nervures intérieures, qui ne sont donc que faiblement sollicitées thermiquement, impliquant notamment de façon avantageuse la possibilité d'utiliser d'autres matériaux que ceux plus coûteux présentant de bonnes propriétés de résistance à la chaleur, sans pour autant nécessiter un surdimensionnement de ces nervures.

De plus, la dilatation thermique du plancher se faisant sensiblement librement vis-à-vis des nervures et des panneaux latéraux, la déformée induite de ces nervures et panneaux est très nettement réduite, ce qui permet au carénage aérodynamique arrière inférieur d'être intégré aux autres structures secondaires du mât, comme la structure arrière.

Enfin, il est noté que la présence des voiles longitudinaux permet de libérer la dilatation thermique du plancher, ce qui a pour effet de diminuer les contraintes mécaniques dans ce dernier. Cette spécificité, ajoutée aux différents effets techniques avantageux exposés ci-dessus, permet d'envisager la réduction de l'épaisseur du plancher par rapport à celle rencontrée dans l'art antérieur, ce qui se traduit en particulier par des gains en termes de masse et de coûts.

Préférentiellement, en section quelconque transversale du carénage, la première extrémité de chaque voile longitudinal et son extrémité latérale associée de plancher de protection thermique forment conjointement une pointe, de préférence en forme de Y. En d'autres termes, chaque voile longitudinal est tel que sa première extrémité latérale est plaquée et au contact de son extrémité latérale de plancher associée, avant de s'écarter progressivement de ce même plancher en allant vers sa seconde extrémité latérale rapportée fixement et directement sur les nervures intérieures.

Cette forme préférée de pointe permet avantageusement de pouvoir conserver une séparation efficace entre le flux primaire circulant sous le plancher et le flux secondaire épousant les panneaux latéraux, de sorte que ces derniers ne sont pas exposés à la chaleur importante du flux primaire.

De préférence, toujours en section quelconque transversale du carénage, chaque voile longitudinal prend sensiblement la forme d'une ligne droite, et le plancher de protection thermique prend sensiblement la forme d'une ligne formant courbure s'ouvrant vers l'extérieur par rapport au carénage, cette dernière forme étant parfaitement adaptée pour assurer un bon écoulement aérodynamique du flux primaire.

Toujours de manière à minimiser les perturbations aérodynamiques et la traînée parasite qui en découle, le plancher de protection thermique est réalisé de manière à ne former qu'une seule pièce.

De préférence, d'une façon analogue, on prévoit que chacun des deux voiles longitudinaux est réalisé de manière à ne former qu'une seule pièce.

A ce titre, il est précisé que chacun des deux voiles longitudinaux et le plancher s'étendent sur une longueur très importante du carénage, de préférence jusqu'à l'amorce de la pyramide aérodynamique arrière, ou bord de fuite, de ce dernier.

Toujours de manière préférentielle, chacun des deux voiles longitudinaux et le plancher de protection thermique sont réalisés en aluminium ou en matériau composite formé par un mélange de résine et de fibres de carbone et/ou de verre, ce qui engendre des gains en termes de masse et de coûts. Néanmoins, on prévoit encore plus préférentiellement qu'ils soient réalisés en titane.

De préférence, la seconde extrémité latérale de chacun des deux voiles longitudinaux est montée fixement sur une portion inférieure des nervures intérieures transversales de rigidification, à distance des panneaux latéraux qui sont quant à eux préférentiellement montés fixement sur des portions latérales de ces mêmes nervures intérieures transversales.

D'une façon plus générale et comme évoqué ci-dessus, le plancher de protection thermique et les deux voiles longitudinaux sont dépourvus de liaison mécanique rigide directe avec les panneaux latéraux du carénage, ce qui veut dire que ces derniers panneaux sont uniquement rapportés fixement indirectement sur le plancher de protection thermique et les deux voiles longitudinaux, en l'occurrence par l'intermédiaire des nervures intérieures transversales. En d'autres termes, cela revient à faire en sorte qu'il existe une coupure mécanique longitudinale entre le plancher et chacun des panneaux latéraux.

Dans cette configuration, le plancher est donc sensiblement libre vis-à-vis des panneaux latéraux, ce qui contribue à faire davantage diminuer les contraintes thermomécaniques subies par celui-ci suite à sa déformation par dilatation thermique.

Ici encore, étant donné que le plancher est susceptible de se déformer par dilatation thermique en rencontrant moins de contraintes qu'auparavant, la qualité aérodynamique de ce plancher est de ce fait également fortement augmentée.

De plus, elle est d'autant plus augmentée que l'absence d'encastrement du plancher dans les panneaux latéraux évite avantageusement la mise en contrainte et la déformation de ces panneaux latéraux qui pourraient être susceptibles de se produire suite à la déformation par dilatation thermique du plancher. A cet égard, il est précisé que les panneaux latéraux baignent dans le flux secondaire relativement froid, de sorte qu'ils ne subissent que très peu de déformation par dilatation thermique. Ainsi, leur niveau global de déformation est donc maintenu relativement bas, ce qui induit une qualité aérodynamique très satisfaisante, participant à la diminution des effets de traînée parasite et à l'amélioration du rapport performance/consommation de l'aéronef.

De préférence, chacun des deux panneaux latéraux est réalisé de manière à ne former qu'une seule pièce.

De même, chacun des deux panneaux latéraux est de préférence réalisé en aluminium ou en matériau composite formé par un mélange de résine et de fibres de carbone et/ou de verre, ou encore en titane.

Selon un mode de réalisation préféré de la présente invention, le carénage comprend en outre deux extensions aérodynamiques de panneaux latéraux chacune associée à l'un des panneaux latéraux qu'elle prolonge en direction du plancher de protection thermique, chaque extension aérodynamique disposant d'une première extrémité rapportée fixement sur son panneau latéral associé, et d'une seconde extrémité coopérant avec l'une des extrémités latérales du plancher de protection thermique contre laquelle elle est uniquement en appui.

Ainsi, aucune liaison rigide n'est prévue entre l'extension aérodynamique et le plancher, de sorte que ce dernier peut continuer à se déformer librement par dilatation. Naturellement, une solution alternative aurait pu être envisagée, dans laquelle chaque panneau latéral s'étend au-delà des nervures intérieures transversales, jusqu'à ce que son extrémité coopère avec l'une des extrémités latérales du plancher de protection thermique, contre laquelle elle est serait toujours uniquement en appui, et non reliée directement et rigidement à celle-ci.

De préférence, chacune des deux extensions aérodynamiques est réalisée de manière à ne former qu'une seule pièce, par exemple en aluminium ou en matériau composite formé par un mélange de résine et de fibres de carbone et/ou de verre, ou encore en titane.

L'invention a aussi pour objet un dispositif d'accrochage d'un moteur destiné à être interposé entre une voilure d'aéronef et le moteur, ce dispositif comprenant un carénage aérodynamique arrière inférieur tel que décrit ci-dessus.

En outre, l'invention a également pour objet un ensemble moteur comprenant un moteur tel qu'un turboréacteur et un dispositif d'accrochage de ce moteur, ce dispositif étant conforme à celui qui vient d'être évoqué.

Enfin, un autre objet de la présente invention est un aéronef comprenant au moins un tel ensemble moteur.

D'autres avantages et caractéristiques de l'invention apparaîtront dans la description détaillée non limitative ci-dessous.

### BRÈVE DESCRIPTION DES DESSINS

Cette description sera faite au regard des dessins annexés parmi lesquels ;
- la figure 1 représente une vue schématique de côté d'un ensemble moteur pour aéronef, comprenant un dispositif d'accrochage selon un mode de réalisation préféré de la présente invention ;
- la figure 2 représente une vue partielle en perspective du carénage aérodynamique arrière inférieur équipant le dispositif d'accrochage montré sur la figure 1, ce carénage étant également objet de la présente invention ;
- la figure 3 représente une vue en perspective d'une partie inférieure du carénage aérodynamique arrière inférieur montré sur la figure 2, intégrant le plancher de protection thermique et ses voiles longitudinaux de raccordement associés ;
- la figure 4 représente une vue en coupe transversale prise le long de la ligne IV-IV de la figure 2 ; et
- la figure 5 représente une vue partielle en coupe transversale prise le long de la ligne V-V de la figure 2.

### EXPOSÉ DÉTAILLÉ DE MODES DE RÉALISATION PRÉFÉRÉS

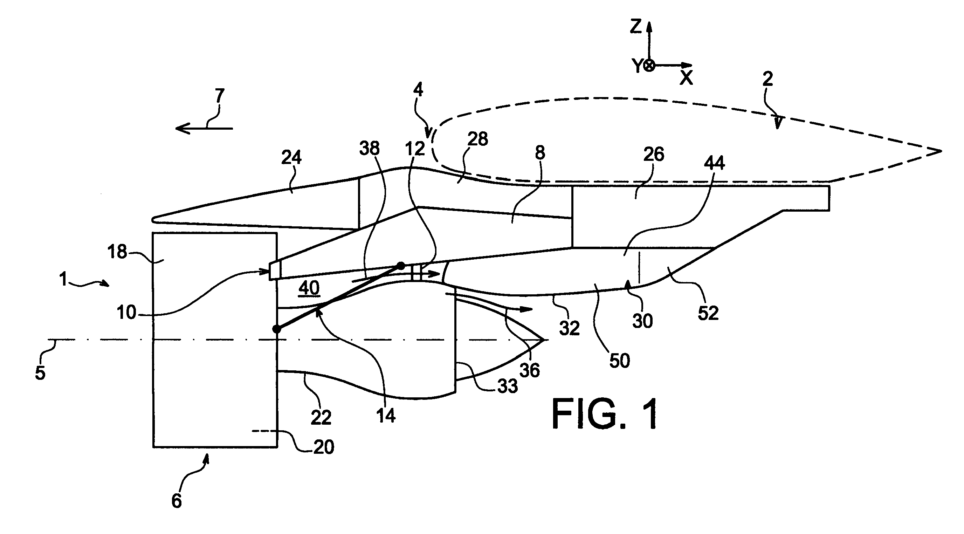

En référence à la figure 1, on voit un ensemble moteur 1 pour aéronef destiné à être fixé sous une aile 2 de cet aéronef, cet ensemble 1 comportant un dispositif d'accrochage 4 selon un mode de réalisation préféré de la présente invention, ainsi qu'un moteur 6 tel qu'un turboréacteur accroché sous ce dispositif 4.

Globalement, le dispositif d'accrochage 4 comporte une structure rigide 8, également appelée structure primaire, portant des moyens d'accrochage du moteur 6, ces moyens d'accrochage disposant d'une pluralité d'attaches moteur 10, 12, ainsi que d'un dispositif de reprise des efforts de poussée 14 générés par le moteur 6.

A titre indicatif, il est noté que l'ensemble 1 est destiné à être entouré d'une nacelle (non représentée), et que le dispositif d'accrochage 4 comporte une autre série d'attaches (non représentées) rapportées sur la structure rigide 8 et permettant d'assurer la suspension de cet ensemble 1 sous la voilure 2 de l'aéronef.

Dans toute la description qui va suivre, par convention, on appelle X la direction longitudinale du dispositif 4 qui est également assimilable à la direction longitudinale du turboréacteur 6 et à celle du carénage aérodynamique arrière inférieur qui sera présenté ci-après, cette direction X étant parallèle à un axe longitudinal 5 de ce turboréacteur 6. D'autre part, on appelle Y la direction orientée transversalement par rapport au dispositif 4 et également assimilable à la direction transversale du turboréacteur 6 et à celle du carénage aérodynamique arrière inférieur, et Z la direction verticale ou de la hauteur, ces trois directions X, Y et Z étant orthogonales entre-elles.

D'autre part, les termes « avant » et « arrière » sont à considérer par rapport à une direction d'avancement de l'aéronef rencontrée suite à la poussée exercée par le turboréacteur 6, cette direction étant représentée schématiquement par la flèche 7.

Sur la figure 1, on peut donc voir les deux attaches moteur 10, 12, le dispositif de reprise des efforts de poussée 14, la structure rigide 8 du dispositif d'accrochage 4, ainsi qu'une pluralité de structures secondaires rapportées sur la structure rigide 8. Ces structures secondaires assurant la ségrégation et le maintien des systèmes tout en supportant des éléments de carénage aérodynamique seront décrites ci-après.

Il est indiqué que le turboréacteur 6 dispose à l'avant d'un carter de soufflante 18 de grande dimension délimitant un canal annulaire de soufflante 20, et comporte vers l'arrière un carter central 22 de plus petite dimension, renfermant le coeur de ce turboréacteur. Les carters 18 et 22 sont bien entendu solidaires l'un de l'autre.

Comme on peut l'apercevoir sur la figure 1, les attaches moteur 10, 12 du dispositif 4 sont prévues au nombre de deux, et respectivement dénommées attache moteur avant et attache moteur arrière.

Dans ce mode de réalisation préféré de la présente invention, la structure rigide 8 prend la forme d'un caisson s'étendant de l'arrière vers l'avant, sensiblement selon la direction X.

Le caisson 8 prend alors la forme d'un mât de conception similaire à celle habituellement observée pour les mâts d'accrochage de turboréacteurs, notamment en ce sens qu'il est pourvu de nervures transversales (non représentées) prenant chacune la forme d'un rectangle orienté dans un plan YZ.

Les moyens d'accrochage de ce mode de réalisation préféré comportent tout d'abord l'attache moteur avant 10 interposée entre une extrémité avant de la structure rigide 8 également appelée pyramide, et une partie supérieure du carter de soufflante 18. L'attache moteur avant 10 est conçue de manière classique et connue de l'homme du métier.

D'autre part, l'attache moteur arrière 12, également réalisée de façon classique et connue de l'homme du métier, est quant à elle interposée entre la structure rigide 8 et le carter central 22.

Toujours en référence à la figure 1, on compte parmi les structures secondaires du mât 4 une structure aérodynamique avant 24, une structure aérodynamique arrière 26, un carénage de raccordement 28 des structures aérodynamiques avant et arrière, et un carénage aérodynamique arrière inférieur 30.

Globalement, ces structures secondaires sont des éléments classiques identiques ou similaires à ceux rencontrés dans l'art antérieur, et connus de l'homme du métier, à l'exception du carénage aérodynamique arrière inférieur 30 qui sera détaillé ci-dessous.

Plus précisément, la structure aérodynamique avant 24 est placée dans le prolongement avant inférieur de la voilure 2 et au-dessus de la structure primaire 8. Elle est montée fixement sur la structure rigide 8, et présente une fonction de profil aérodynamique entre une partie supérieure des capots de soufflante articulés sur celle-ci, et le bord d'attaque de la voilure. Cette structure aérodynamique avant 24 dispose alors non seulement d'une fonction de carénage aérodynamique, mais permet également la mise en place, la ségrégation et le cheminement de différents systèmes (air, électrique, hydraulique, carburant). De plus, la partie avant de cette structure 24 n'étant pas au contact de la structure rigide 8, il est habituellement interposé un échangeur thermique dans l'espace défini entre ces deux éléments.

Directement dans le prolongement arrière de cette structure 24, toujours sous la voilure et monté au-dessus de la structure rigide 8, se trouve le carénage de raccordement 28, également appelé « karman ». Ensuite, toujours vers l'arrière, le carénage de raccordement 28 est prolongé par la structure aérodynamique arrière 26, qui contient une partie des équipements du mât. Cette structure 26 est de préférence située entièrement en arrière par rapport à la structure rigide 8, et est donc attachée sous la voilure de l'aéronef.

Enfin, sous la structure rigide 8 et la structure aérodynamique arrière 26, se trouve le carénage aérodynamique arrière inférieur 30, également appelé « bouclier » ou « Aft Pylon Fairing ». Ses fonctions essentielles sont la formation d'une barrière thermique également dite anti-feu servant à protéger le mât et la voilure de la chaleur dégagée par le flux primaire, et la formation d'une continuité aérodynamique entre la sortie du moteur et le mât d'accrochage.

De manière connue de l'homme du métier, le carénage 30 précité comporte un plancher de protection thermique 32 pourvu d'une surface extérieure destinée à être épousée par un flux primaire du moteur qu'il délimite partiellement radialement vers l'extérieur, ce flux primaire s'échappant de la tuyère 33 du moteur étant représenté schématiquement par la flèche 36. Par ailleurs, le carénage 30 comporte aussi deux panneaux latéraux 44 qui sont quant à eux prévus pour être épousés extérieurement par un flux secondaire du moteur représenté schématiquement par la flèche 38, en raison de leur implantation dans le canal annulaire 40 de flux secondaire du moteur, et/ou en sortie de celui-ci.

Il est noté que dans le mode de réalisation préféré décrit où le moteur 6 est destiné à être suspendu sous la voilure de l'aéronef, le plancher 32 de protection thermique du mât et de la voilure vis-à-vis du flux primaire 36, constitue une portion inférieure du carénage 30. Naturellement, ce plancher constituerait une portion supérieure du carénage dans le cas alternatif où le moteur serait destiné à être implanté au-dessus de la voilure.

Enfin, comme cela est visible sur la figure 1, il est prévu que l'extrémité avant du plancher 32 vienne épouser l'extrémité arrière supérieure de la tuyère 33, ou bien qu'elle soit fortement rapprochée de cette même extrémité arrière de tuyère 33.

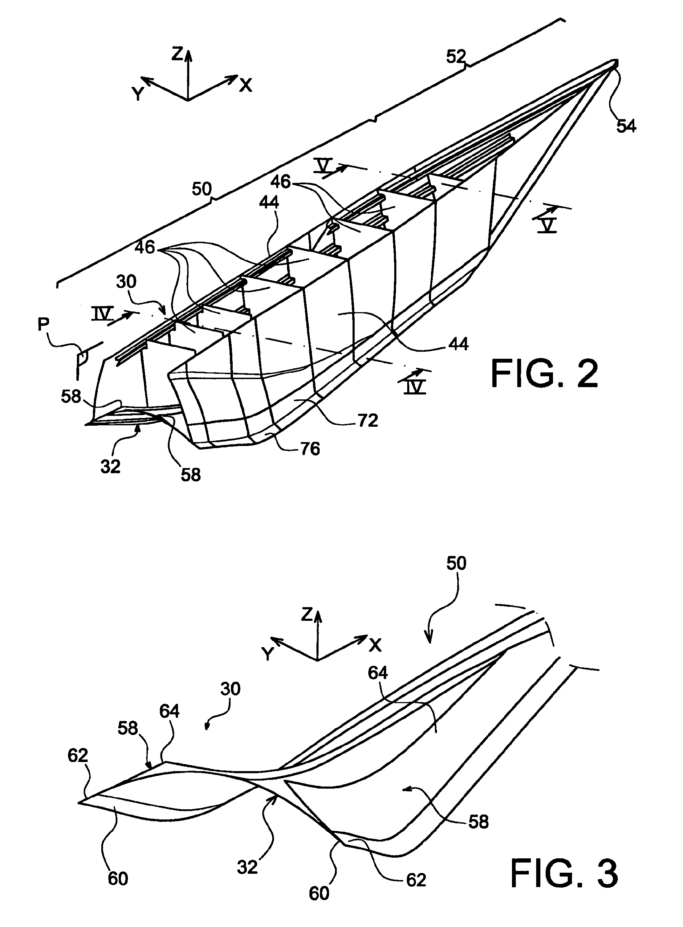

En référence à présent aux figures 2 à 5, on peut apercevoir de façon plus détaillée le carénage aérodynamique arrière inférieur 30, qui prend la forme générale d'un caisson ouvert vers le haut, c'est-à-dire en direction des autres structures du mât 4 sur lesquelles il est destiné à être monté, à savoir la structure aérodynamique arrière 26 et la structure rigide 8. Le carénage 30 présente de préférence un plan de symétrie P correspondant à un plan XZ, ce plan P constituant également un plan vertical de symétrie pour l'ensemble du dispositif d'accrochage 4, et pour le moteur 6.

En référence plus particulièrement à la figure 2, le carénage aérodynamique arrière inférieur 30 en forme de caisson comprend les deux panneaux latéraux 44 chacun grossièrement orienté dans un plan XZ, de part et d'autre du plan P. Ils sont assemblés entre eux par des nervures intérieures transversales de rigidification 46 espacées les unes des autres selon la direction X, chacune de ces nervures 46 étant orientée selon un plan YZ et prenant par exemple la forme d'un rectangle ou d'un carré. A cet égard, même si cela n'a pas été représenté, le carénage 30 présente également de préférence une nervure de fermeture avant du caisson.

Les panneaux latéraux 44 sont montés fixement et directement sur les portions latérales de chacune des nervures intérieures 46, à l'aide de moyens conventionnels connus de l'homme du métier.

D'autre part, le carénage 30 intègre le plancher de protection thermique 32 en partie inférieure du caisson, la partie supérieure restant de préférence ouverte avant d'être rapportée sur le dispositif d'accrochage, comme cela est bien visible sur la figure 2.

Toujours sur cette même figure, on peut apercevoir que le carénage 30 se décompose en deux portion distinctes mais solidaires l'une de l'autre, à savoir une portion avant 50 constituant la majeure partie du carénage, par exemple 60 à 85 % de celui-ci en terme de longueur selon la direction X, et une faible portion arrière 52 prenant globalement la forme d'une pyramide ou d'une pointe dont la base et reliée rigidement à la portion avant 50, et dont le sommet 54 constitue une extrémité arrière du carénage 30. A titre indicatif, la portion avant 50 présente une section transversale grossièrement homogène sur toute sa longueur.

Les panneaux latéraux 44 s'étendent de préférence chacun d'une seule pièce d'un bout à l'autre du carénage 30, c'est-à-dire à la fois le long de la portion avant 50, et le long de la portion arrière 52. En revanche, le plancher de protection thermique 32 s'étend lui de préférence d'une seule pièce uniquement sur la portion avant 50, et non sur la portion arrière 52, même si cela pourrait bien entendu être envisagé, sans sortir du cadre de l'invention. Cette particularité s'explique notamment par le fait que la portion arrière 52 en forme de pyramide s'éloigne progressivement de l'axe du moteur, de sorte que le flux primaire, qui perd de toute façon en intensité de chaleur en allant vers l'arrière, provoque une incidence thermique moindre sur l'élément inférieur de fermeture de la pyramide 52.

En outre, il est indiqué que le fait de prévoir chacun des éléments mentionnés ci-dessus d'une seule pièce n'exclut pas la possibilité de les fabriquer à l'aide de plusieurs portions distinctes rapportées fixement les unes aux autres, comme par exemple plusieurs portions se succédant selon la direction X. Cela vaut également pour les éléments suivants qui seront décrits comme étant susceptibles d'être fabriqués d'une seule pièce.

L'une des particularités de la présente invention réside dans le fait que le plancher 32 est déporté vers le bas par rapport aux nervures intérieures transversales 46, à l'aide de deux voiles longitudinaux de raccordement 58 rapportés fixement et directement sur des extrémités latérales de ce plancher 32, comme cela va maintenant être détaillé en référence aux figures 3 à 5.

En effet, sur la figure 3, on peut voir que chaque voile longitudinal 58 présente une première extrémité latérale 62, ou extrémité latérale inférieure, qui est montée fixement et directement sur l'une des extrémités latérales 60 du plancher 32, par exemple par rivetage ou moyen similaire.

Ainsi, on fait de préférence en sorte que chacune des deux jonctions mécaniques rigides et directes entre les deux extrémités 60 et 62 s'effectue tout le long de la portion avant 50 du carénage, en suivant grossièrement la direction X.

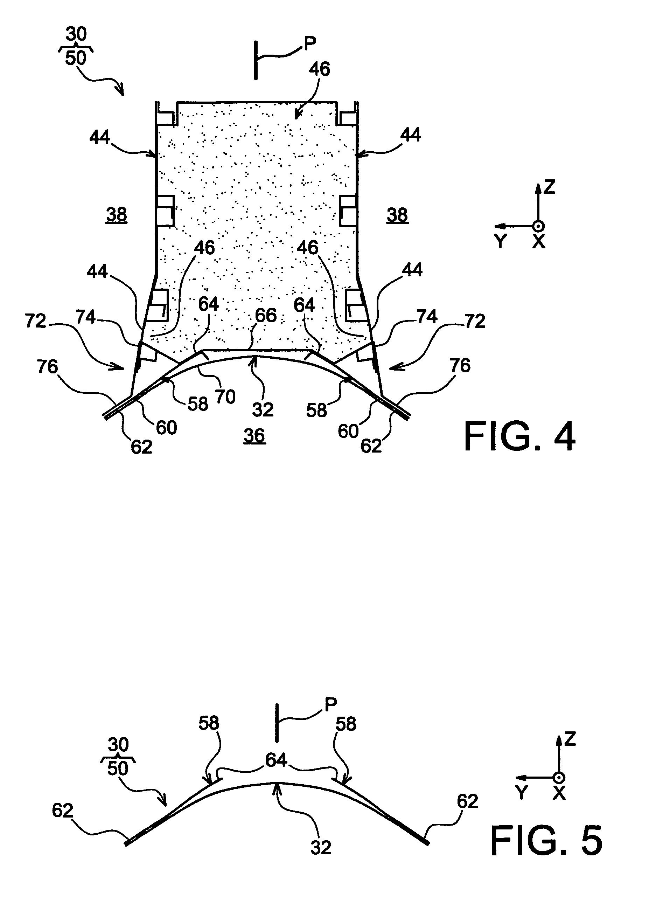

Les deux voiles longitudinaux 58 qui s'étendent au-dessus du plancher 32 sont également de préférence chacun réalisés de manière à ne former qu'une seule pièce, et s'étendent depuis leur extrémité inférieure 62 vers une seconde extrémité latérale 64 ou extrémité latérale supérieure, dont la fonction est de se rapporter rigidement sur les nervures intérieures du carénage, comme cela est le mieux visible sur la figure 4 montrant une vue en coupe prise entre deux nervures consécutives.

En effet, on peut apercevoir sur cette figure 4 que le plancher de protection thermique 32 est rapporté indirectement sur la portion inférieure 66 des nervures intérieures 46 par l'intermédiaire des deux voiles 58, qui constituent d'ailleurs les seuls moyens assurant le montage du plancher 32 sur ces mêmes nervures.

Pour ce faire, la seconde extrémité latérale 64 de chacun des deux voiles 58, qui se trouve sensiblement écartée du plancher 32 déporté vers le bas, est montée fixement et directement sur la portion inférieure 66 des nervures intérieures 46, dans une position écartée des panneaux latéraux 44. Par conséquent, le plancher 32 n'est plus monté directement sur les nervures intérieures comme cela était le cas antérieurement, ce qui lui permet avantageusement de se déformer plus librement par dilatation thermique suite à l'importante chaleur dégagée par le flux primaire 36 épousant ce plancher 32.

A cet égard, il est rappelé que le plancher de protection thermique 32 est pourvu d'une surface extérieure référencée 70 sur la figure 4, cette surface étant destinée à être épousée par le flux primaire 36 qu'elle délimite en partie radialement vers l'extérieur, alors que les panneaux latéraux 44 sont quant à eux prévus pour être épousés extérieurement par le flux secondaire 38.

Pour conserver une séparation efficace entre le flux primaire 36 circulant sous le plancher 32 et le flux secondaire 38 épousant les panneaux latéraux 44, c'est-à-dire pour éviter que le flux primaire de température très élevée ne remonte et se propage le long de ces panneaux latéraux 44, on fait de préférence en sorte qu'en section quelconque transversale du carénage, la première extrémité 62 de chaque voile longitudinal 58 et son extrémité latérale associée de plancher 60 forment conjointement une pointe en forme de Y. Plus précisément, comme cela a été représenté, le Y est disposé de manière à ce que sa pointe, c'est-à-dire sa portion constituée par la zone de contact entre les deux extrémités 60, 62, soit orientée sensiblement vers le bas et latéralement vers l'extérieur par rapport au carénage 30, afin de correctement circonscrire le flux primaire 36 dans la partie inférieure du carénage, à savoir le long et au contact du plancher de protection thermique 32.

A ce titre, toujours en section quelconque transversale du carénage, on prévoit que chaque voile longitudinal 58 prend sensiblement la forme d'une ligne droite inclinée de manière à se rapprocher d'un centre du carénage en allant vers sa seconde extrémité latérale 64, tandis que le plancher de protection thermique 32 situé en dessous de ces voiles 58 prend quant à lui sensiblement la forme d'une ligne formant courbure s'ouvrant vers l'extérieur par rapport au carénage 30, toujours dans le but de circonscrire le flux primaire 36 dans la partie inférieure de ce carénage.

Avec cette géométrie particulière, il est donc aisément possible de faire en sorte que le plancher 32 soit entièrement déporté et dépourvu de contact vis-à-vis des nervures 46, un espace libre étant donc prévu entre la portion inférieure 66 de celles-ci et le plancher 32.

Enfin, il est noté que le plancher 32, monté fixement et directement à ses deux extrémités latérales 30 sur les deux voiles agencés symétriquement par rapport au plan P précité, peut présenter une courbure s'aplatissant progressivement en allant vers l'arrière, comme le fait comprendre la figure 5, correspondant à une coupe transversale effectuée plus en arrière, sur laquelle le plancher 32 adopte en effet une courbure de plus grand diamètre.

Etant donné que le plancher de protection thermique 32 et les deux voiles longitudinaux de raccordement 58 sont dépourvus de liaison mécanique rigide directe avec les panneaux latéraux 44, et de préférence situés plus bas qu'une extrémité inférieure de ces panneaux, le carénage 32 est de préférence équipé de moyens additionnels permettant d'effectuer une jonction aérodynamique entre l'extrémité inférieure de chaque panneau latéral 44 et sa pointe de Y associée située en regard et à distance de celle-ci, formée par les extrémités latérales 60, 62 se recouvrant.

Pour ce faire, il est prévu deux extensions aérodynamiques de panneaux latéraux 72, disposées symétriquement par rapport au plan P et chacune associée à l'un des deux panneaux latéraux 44 qu'elle prolonge en direction du plancher de protection thermique 32, et plus précisément en direction du Y formé conjointement par ce dernier et par le voile longitudinal associé 58.

De préférence, chaque extension aérodynamique 72 dispose d'une première extrémité 74 ou extrémité supérieure rapportée fixement et directement sur son panneau latéral associé 44, et d'une seconde extrémité ou extrémité basse 76 coopérant avec son extrémité latérale de plancher associée 60, contre laquelle elle est uniquement en appui. De préférence, les deux liaisons réalisées aux deux extrémités de chaque extension 72, à savoir la liaison rigide et la liaison d'appui simple, s'étendent de façon continue sur toute la portion avant 50 du carénage. A ce titre, ici encore, chacune des extensions 72 s'étend de préférence d'une seule pièce uniquement sur la portion avant 50, et non sur la portion arrière 52, même si cela pourrait bien entendu être envisagé, sans sortir du cadre de l'invention. D'une manière générale, il est noté que les extensions aérodynamiques 72, les voiles de raccordement 58 et le plancher de protection thermique 32 s'étendent préférentiellement sur une même longueur selon la direction X.

En section quelconque transversale du carénage, chaque extension 72 prend la forme grossière d'un L dont la base constituée par l'extrémité basse 76 est en appui contre la pointe du Y formée par les extrémités en contact 60, 62 du plancher et du voile associé. Ainsi, on fait de préférence en sorte que cette extrémité basse 76 soit en appui contre le plancher 32 sans être directement au contact de celui-ci, mais au contact du voile de raccordement 58. La liaison d'appui simple recherchée, permettant la libre dilatation thermique du plancher 32, prend la forme d'un contact surfacique s'étendant donc tout le long de la portion avant 50 du carénage.

En outre, l'extrémité de la branche longue du L, sensiblement orientée selon la direction Z, constitue quant à elle l'extrémité supérieure 74 de l'extension 72. Elle est montée fixement sur l'extrémité inférieure du panneau latéral 44 qui peut éventuellement s'étendre légèrement vers le bas au-delà des nervures intérieures 46, comme montré sur la figure 4, la fixation s'effectuant de préférence à l'aide de moyens conventionnels du type rivets ou moyens similaires.

Enfin, en raison de la conception particulière du carénage 30 qui vient d'être détaillée, l'ensemble des éléments constitutifs de ce dernier peuvent être réalisés en aluminium ou en matériau composite formé par un mélange de résine et de fibres de carbone et/ou de verre, ce qui engendre avantageusement une réduction de sa masse et de son coût de fabrication.

Bien entendu, diverses modifications peuvent être apportées par l'homme du métier à l'invention qui vient d'être décrite, uniquement à titre d'exemples non limitatifs. A cet égard, on peut notamment indiquer qui si l'ensemble moteur 1 a été présenté dans une configuration adaptée pour qu'il soit suspendu sous la voilure de l'aéronef, cet ensemble 1 pourrait également se présenter dans une configuration différente lui permettant d'être monté au-dessus de cette même voilure.

## Revendications

1. Carénage aérodynamique arrière inférieur (30) pour dispositif d'accrochage (4) d'un moteur (6) destiné à être interposé entre une voilure (2) d'aéronef et ledit moteur (6), ledit carénage formant caisson comprenant deux panneaux latéraux (44) assemblés entre eux par des nervures intérieures transversales de rigidification (46) espacées les unes des autres selon une direction longitudinale (X) dudit carénage, et comportant en outre un plancher de protection thermique (32) pourvu d'une surface extérieure (70) destinée à être épousée par un flux primaire (36) dudit moteur, ledit plancher de protection thermique (32) présentant deux extrémités latérales opposées (60, 60),
**caractérisé en ce qu'**il comporte en outre deux voiles longitudinaux de raccordement (58) déportant ledit plancher de protection thermique (32) desdites nervures intérieures transversales de rigidification (46), lesdits deux voiles longitudinaux (58) disposant chacun d'une première extrémité latérale (62) montée fixement respectivement sur l'une et l'autre des deux extrémités latérales (60, 60) dudit plancher de protection thermique (32), et d'une seconde extrémité latérale (64) montée fixement sur lesdites nervures intérieures transversales de rigidification (46).

2. Carénage aérodynamique selon la revendication 1, **caractérisé en ce qu'**en section quelconque transversale dudit carénage, ladite première extrémité (62) de chaque voile longitudinal (58) et son extrémité latérale associée (60) de plancher de protection thermique (32) forment conjointement une pointe.

3. Carénage aérodynamique selon l'une quelconque des revendications précédentes, **caractérisé en ce que** ledit plancher de protection thermique (32) est réalisé de manière à ne former qu'une seule pièce.

4. Carénage aérodynamique selon l'une quelconque des revendications précédentes, **caractérisé en ce que** chacun des deux voiles longitudinaux (58) est réalisé de manière à ne former qu'une seule pièce.

5. Carénage aérodynamique selon l'une quelconque des revendications précédentes, **caractérisé en ce que** ladite seconde extrémité latérale (64) de chacun des deux voiles longitudinaux (58) est montée fixement sur une portion inférieure (66) desdites nervures intérieures transversales de rigidification (46), à distance desdits panneaux latéraux (44).

6. Carénage aérodynamique selon l'une quelconque des revendications précédentes, **caractérisé en ce que** ledit plancher de protection thermique (32) et lesdits deux voiles longitudinaux (58) sont dépourvus de liaison mécanique rigide directe avec lesdits panneaux latéraux (44) du carénage.

7. Carénage aérodynamique selon l'une quelconque des revendications précédentes, **caractérisé en ce que** ledit plancher de protection thermique (32) est déporté desdites nervures intérieures transversales (46) de manière à être entièrement dépourvu de contact avec celles-ci.

8. Dispositif d'accrochage (4) d'un moteur (6) destiné à être interposé entre une voilure (2) d'aéronef et ledit moteur (6), **caractérisé en ce qu'**il comprend un carénage aérodynamique arrière inférieur (30) selon l'une quelconque des revendications précédentes.

9. Ensemble moteur (1) comprenant un moteur (6) et un dispositif d'accrochage (4) du moteur (6) selon la revendication 8.

10. Aéronef comprenant au moins un ensemble moteur (1) selon la revendication 9.

## Claims

1. Aft pylon fairing (30) for a suspension system (4) for an engine (6), designed to be inserted between an aircraft wing (2) and said engine (6), said fairing forming a box comprising two side panels (44) assembled to each other by inner cross stiffening ribs (46) spaced at intervals from each other along a longitudinal direction (X) of said fairing, and also comprising a heat protection deck (32) provided with an outer surface (70) designed to delimit a core engine flow (36) of said engine, said heat protection deck (32) having two opposite side ends (60, 60),
**characterised in that** it also comprises two longitudinal connecting walls (58) offsetting said heat protection deck (32) from said inner cross stiffening ribs (46), each of said two longitudinal walls (58) having a first side end (62) fixed to one or the other of the two side ends (60, 60) of said heat protection deck (32), and a second side end (64) rigidly fixed to said inner cross stiffening ribs (46).

2. Aerodynamic fairing according to claim 1, **characterised in that** in any arbitrary cross-section of said fairing, said first end (62) of each longitudinal wall (58) and its associated side end (60) of the heat protection deck (32) work together to form a tip.

3. Aerodynamic fairing according to anyone of the preceding claim, **characterised in that** said heat protection deck (32) is made so as to form a single piece.

4. Aerodynamic fairing according to any one of the preceding claims, **characterised in that** each of the two longitudinal walls (58) is made so as to form a single piece.

5. Aerodynamic fairing according to any one of the preceding claims, **characterised in that** said second side end (64) of each of the two longitudinal walls (58) is mounted fixed on a lower portion (66) of said inner stiffening cross ribs (46), at a distance from said side panels (44).

6. Aerodynamic fairing according to any one of the preceding claims, **characterised in that** said heat protection deck (32) and said two longitudinal walls (58) do not have any rigid direct mechanical link with said side panels (44) of the fairing.

7. Aerodynamic fairing according to any one of the preceding claims, **characterised in that** said heat protection deck (32) is offset from said inner cross ribs (46) such that it has no contact with the ribs.

8. Suspension system (4) for an engine (6) designed to be inserted between an aircraft wing (2) and said engine (6), **characterised in that** it comprises an aft pylon fairing (30) according to any one of the preceding claims.

9. Engine assemble (1) comprising an engine (6) and a suspension system (4) for the engine (6) according to claim 8.

10. Aircraft comprising at least one engine assembly (1) according to claim 9.

## Patentansprüche

1. Hintere untere aerodynamische Verkleidung (30) für eine Befestigungsvorrichtung (4) eines Triebwerks (6) zur Anbringung zwischen einer Tragfläche (2) eines Luftfahrzeugs und dem Triebwerk (6), wobei die Verkleidung einen Kasten bildet, der zwei Seitenplatten (44) umfasst, die über innenliegende Versteifungsquerrippen (46), die voneinander längs einer Längsrichtung (X) der Verkleidung beabstandet sind, untereinander verbunden sind und ferner einen Wärmeschutzboden (32) aufweist, der mit einer äußeren Oberfläche (70) ausgestattet ist, der ein Primärstrom (36) des Triebwerks folgen soll, wobei der Wärmeschutzboden (32) zwei entgegengesetzte seitliche Enden (60, 60) aufweist,
**dadurch gekennzeichnet, dass** sie ferner zwei Längsflügel zur Befestigung (58), die den Wärmeschutzboden (32) von den innenliegenden Versteifungsquerrippen (46) wegverlagern, aufweist, wobei die zwei Längsflügel (58) jeweils ein erstes seitliches Ende (62), das fest an dem einen bzw. dem anderen der beiden seitlichen Enden (60, 60) des Wärmeschutzbodens (32) angebracht ist, und ein zweites seitliches Ende (64), das fest an den innenliegenden Versteifungsquerrippen (46) angebracht ist, aufweisen.

2. Aerodynamische Verkleidung nach Anspruch 1, **dadurch gekennzeichnet, dass** in einem beliebigen Querschnitt der Verkleidung das erste Ende (62) jedes Längsflügels (58) und das hierzu entsprechende seitliche Ende (60) des Wärmeschutzbodens (32) gemeinsam eine Spitze bilden.

3. Aerodynamische Verkleidung nach einem der vorhergehenden Ansprüche, **dadurch gekennzeichnet, dass** der Wärmeschutzboden (32) derart ausgebildet ist, dass er nur ein einziges Teil bildet.

4. Aerodynamische Verkleidung nach einem der vorhergehenden Ansprüche, **dadurch gekennzeichnet, dass** jeder der beiden Längsflügel (58) derart ausgeführt ist, dass er nur ein einziges Teil bildet.

5. Aerodynamische Verkleidung nach einem der vorhergehenden Ansprüche, **dadurch gekennzeichnet, dass** das zweite seitliche Ende (64) jedes der beiden Längsflügel (58) fest an einem unteren Teil (66) der innenliegenden Versteifungsquerrippen (46) mit einem Abstand von den Seitenplatten (44) angebracht ist.

6. Aerodynamische Verkleidung nach einem der vorhergehenden Ansprüche, **dadurch gekennzeichnet, dass** der Wärmeschutzboden (32) und die beiden Längsflügel (58) keine starre mechanische Verbindung mit den Seitenplatten (44) der Verkleidung aufweisen.

7. Aerodynamische Verkleidung nach einem der vorhergehenden Ansprüche, **dadurch gekennzeichnet, dass** der Wärmeschutzboden (32) von den innenliegenden Querrippen (46) derart wegverlagert ist, dass überhaupt kein Kontakt mit diesen besteht.

8. Befestigungsvorrichtung (4) für ein Triebwerk (6) zur Anbringung zwischen einer Tragfläche (2) eines Luftfahrzeugs und dem Triebwerk (6), **dadurch gekennzeichnet, dass** sie eine hintere untere aerodynamische Verkleidung (30) nach einem der vorhergehenden Ansprüche umfasst.

9. Triebwerkanordnung (1), umfassend ein Triebwerk (6) und eine Befestigungsvorrichtung (4) des Triebwerks (6) nach Anspruch 8.

10. Luftfahrzeug, umfassend mindestens eine Triebwerkanordnung (1) nach Anspruch 9.
